# EUROPEAN PATENT SPECIFICATION

(11) **EP 0 736 029 B1**
(45) Date of publication and mention of the grant of the patent: **08.03.2006**
(21) Application number: 95904333.2
(22) Date of filing: 15.12.1994
(51) Int. Cl.: C07D 493/04, C07D 497/04, A61K 31/34, C07D 317/00, C07D 327/00

(54) **ANTICONVULSANT PSEUDOFRUCTOPYRANOSE SULFAMATES**
ANTIKONVULSIVE PSEUDOFRUCTOPYRANOSE SULFAMATE
SULFAMATES DE PSEUDOFRUCTOPYRANOSE ANTICONVULSIFS

(30) Priority: 23.12.1993 US 173399; 10.11.1994 US 337597
(43) Date of publication of application: 09.10.1996
(73) Proprietor: Ortho-McNeil Pharmaceutical, Inc., Raritan, NJ 08869-0606 (US)
(72) Inventor: COSTANZO, Michael, J., Ivyland, PA 18974 (US); MARYANOFF, Bruce, E., New Hope, PA 18938 (US); McCOMSEY, David, F., Warminster, PA 18974 (US); NORTEY, Samuel, O., LaMott, PA 19126 (US)
(74) Representative: Mercer, Christopher Paul
(86) International application number: PCT/US1994/014591
(87) International publication number: WO 1995/017406

(56) References cited:
- EP-A- 0 138 441
- US-A- 5 242 942
- JOURNAL OF ORGANIC CHEMISTRY., vol.59, no.9, 1994, EASTON US pages 2652 - 2654 D. F. MCCOMSEY ET AL 'Improved synthesis of pseudo-beta-D-fructopyranose, a carbocyclic monosaccharide from (-)-quinic acid'

## Description

### BACKGROUND OF THE INVENTION

Sulfamates of various structures, including those derived from monosaccharides, are described in *J*. *Med. Chem.* **1987**, 30, 880 and in U.S, Patent No. 4,075,351. Certain of these sulfamates are useful as pharmaceutical agents. More recently, sulfamates having various pharmaceutical activity in the areas of epilepsy, glaucoma, peptic ulcers, and male infertility are described in U.S. Patents Nos. 4,513,006, 4,459,601 and 4,792,569. One of the compounds covered by U.S. Patent 4,513,006, topiramate, has not only been found to exhibit particularly significant anticonvulsant activity in animals, but also appears to be useful in humans for the treatment of epilepsy (*Drugs Future* **1989**,14,342),

U.S. 4,513,006 corresponds to EP-A-138441 and discloses sulfamates of the following formula: 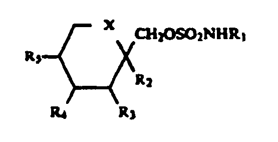
wherein X is CH₂ or oxygen; R₁ is hydrogen or alkyl; and R₂, R₃, R₄ and R₅ are independently hydrogen or lower alkyl and, when X is CH₂, and R₄ and R₅ may be alkene groups joined to form a benzene ring and, when X is oxygen, R₂ and R₃ and/or R₄ and R₅ together may be a methylenedioxy group of the following formula: 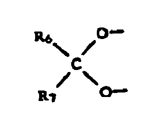
wherein R₆ and R₇ are the same or different and are hydrogen, lower alkyl or are alkyl and are joined to form a cyclopentyl or cyclohexyl ring.

U.S. 5,242,942 discloses sulfamates of the general formula: 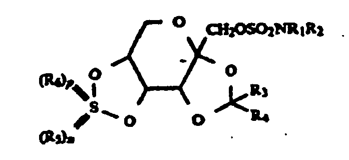
wherein R₁ and R₂ are the same or different and are hydrogen, C₁-C₁₂ alkyl, C₃₋C₁₀ cycloalkyl, allyl, benzyl, CH₂(C₁-C₄ perfluoroalkyl), or are taken together with the N to represent an azido group;
wherein R₃ and R₄ are the same or different and are hydrogen, C₁-C₆ alkyl, or are taken together to form a cyclopentyl or cyclohexyl ring;
R₅ and R₆ may be the same or different and are oxygen or NR₇; wherein R₇ is selected from hydrogen, C₁-C₄ alkyl, C₁-C₄ perfluoralkyl, arenesulfonyl, C₁-C₄ alkoxycarbonyl or benzyloxycarbonyl;
wherein n equals zero or one and p equals zero or one, provided that n and p are not both equal to zero at the same time;
and a pharmaceutically acceptable salt thereof.

While sulfamate compounds of the type disclosed in U.S. Patent No. 4,513,006 have been shown to exhibit useful biological activity when administered to mammals, other compounds with equal or improved activity compared to topiramate would be desirable.

Replacement of the ring oxygen of cyclic monosaccharides by a methylene group affords an interesting class of compounds which has been referred to as "pseudo-sugars". These cyclitol compounds can possess enhanced biological activity compared to the original monosaccharides.

Accordingly, it is an object of the present invention to describe novel pseudo-β-fructopyranose sulfamate derivates, which are related to topiramate, with potent anticonvulsant activity.

### SUMMARY OF THE INVENTION

It has been found that certain sulfamate derivatives represented by the general formula I: 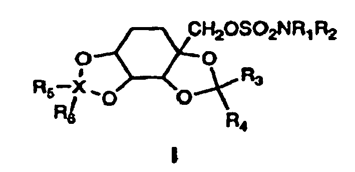
wherein R₁, R₂, R₃, R₄, R₅ and R₆ and X are as defined in claim 1 exhibit anticonvulsant activity. As a result, the compounds and pharmaceutical compositions containing such compounds of the present invention are useful for the treatment of convulsions such as epileptic seizures.

A particularly preferred compound of formula **I** is :
(1R,2R,3S,4S)-(1,2:3,4-di-O-methylethylidenecyclohexan-1,2,3,4-tetraol-4-yl)methyl sulfamate, i.e., where R₁ and R₂ are hydrogen, R₃, R₄, R₅, and R₆ are methyl and X is carbon in formula **I**.

Included within the scope of this invention are the various individual anomers, diastereomers and enantiomers as well as mixtures thereof. Such compounds are included within the definition of formula **I**. In addition, the compounds of this invention include pharmaceutically acceptable salts, for example; alkali metal salts, such as sodium or potassium, ammonium salts, dialkylammonium salts, trialkylammonlum salts, tetraalkylammonium salts, and tromethamine salts. Hydrates and other solvates of the compound of the formula **I** are also included within the scope of this invention.

Compounds of formula **I** may be prepared as outlined in the following schemes.

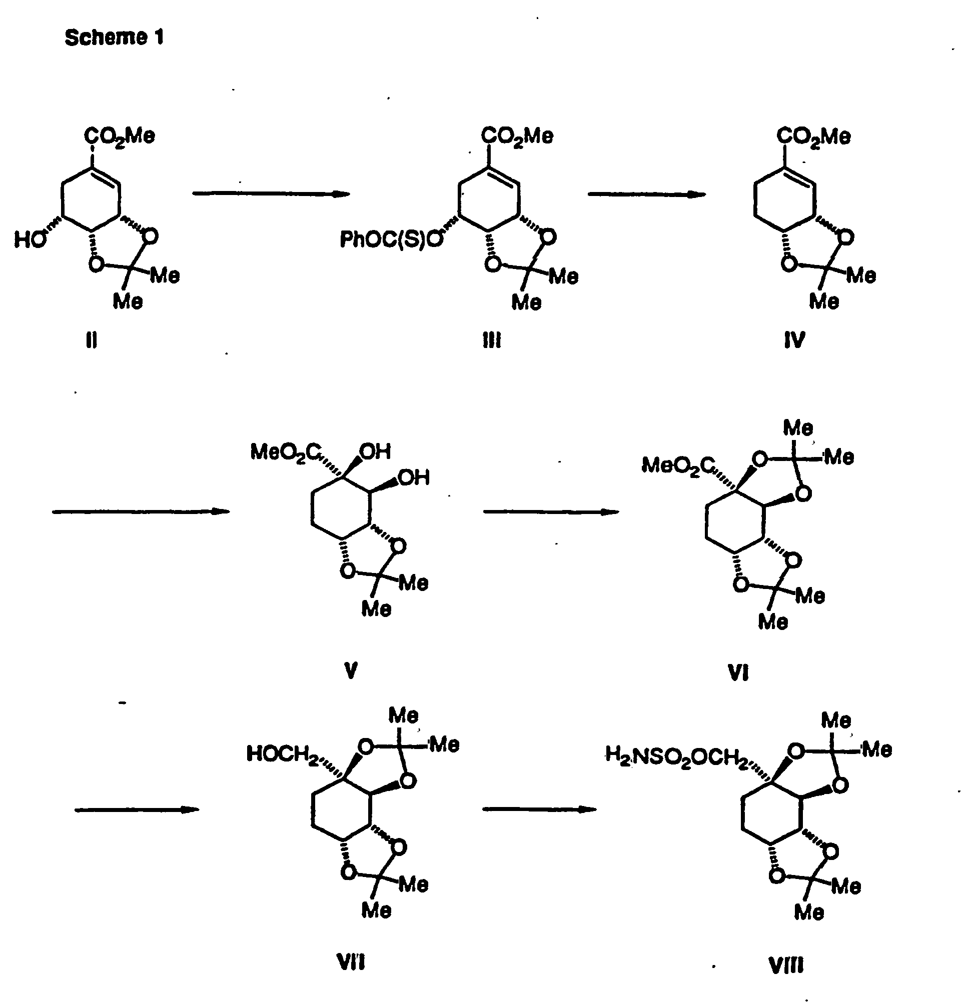

More specifically, the alcohol **II**, wherein R₅ and R₆ are methyl, (prepared according to the method of Shing, T.K. and Tang, Y. *Tetrahedron* **1990**, *46,* 6575-6584) is treated with a mixture of phenyl chlorothionoformate and pyridine and a catalytic amount of 4-dimethylaminopyridine in an appropiate solvent such as methylene chloride for 2 h at room temperature to give thionocarbonate **III** (R₅ = R₆ = Me). This carbonate is reduced with tributyltin hydride under free radical conditions using tert butyl peroxide in toluene at reflux for 2 h to give the ester **IV** (R₅ = R₆ = Me).

The diol V (R₅ = R₆ = Me) is prepared by combining **IV** (R₅ = R₆ = Me) with trimethylamine oxide, pyridine, water, and osmium tetroxide in t-butanol at reflux for 2 h. This diol is treated with 2-methoxypropene and an acid catalyst such as camphorsulfonic acid in a suitable solvent such as methylene chloride at room temperature for 2-6 h to afford bis-acetonide **VI** (R₃, R₄, R₅, R₆ = Me).

Reduction of **VI** (R₃, R₄, R₅, R₆ = Me) with diisobutylaluminum hydride at -20°C to room temperature in a suitable solvent such as tetrahydrofuran gave alcohol **VII** (R₃, R₄, R₅, R₆ = Me). Sulfamate **VIII** (R₁ = R₂ = H; R₃, R₄, R₅, R₆ = Me) was prepared by treating **VII** (R₃, R₄, R₅, R₆ = Me) with sulfamoyl chloride and triethylamine in a suitable solvent such as methylene chloride at 0°C for 2-6 h.

Pharmaceutically acceptable salts of the compounds of formula (1) may be prepared by reacting the sulfamate of formula (I) with an appropriate base and recovering the salt.

The compounds of formula I are particularly useful as anticonvulsant agents in mammals including humans. The anticonvulsant activity of the subject compounds was determined using a standard "maximal electroshock test" (MES). In this test, activity is indicated by a block of the toxic extensor seizure caused by application of an electric shock to mice via corneal electrodes, as described by Swinyard et al. in *J. Pharmacol. Expt. Ther.* **1952**, *106*, 319, and recorded as % block. A more recent description of current anticonvulsant drug screening is given by Swinyard in *Epilepsia* **1978**, *19*, 409.

In the test, albino male CRS-CD1 mice weighing between 18-25 g were used in all experiments (obtained from Charles River). They were allowed food and water *ad libitum* and were used only once. The electroshock apparatus and the corneal electrodes were purchased from Wahlquist Instrument Company, Salt Lake City, Utah.

Maximal electroshock seizures were Induced by the delivery of a 60 Hertz (Hz) current of 50 milliamps (mA) intensity to the mouse through corneal electrodes for 0.2 seconds as originally described by Swinyard (1952). This stimulus intensity is approximately 4 to 6 times the current producing 100% tonic extensor convulsions. During the validation of the MES test, the duration of the various seizure components following maximal electroshock was measured as follows: hindleg tonic flexion was measured from the time of the application of the stimulus to the time of onset of hindleg tonic extension (i.e. when the hindlegs deviate by greater than an angle of 90° from the torso), hindleg tonic extensor was measured from the time of extensor thrust to the onset of generalized clonus, and terminal clonus was measured from the beginning to the end of bilateral rhythmic clonic jerking. Mortality was also recorded. The duration of each seizure component agreed well with the values previously reported by Tedeschi *et al.* in *J. Pharmacol. Expt. Ther.* **1955**,*116*, 107. The corneal electrodes were concave so that saline could be applied to the electrodes to reduce mortality. If this procedure is followed, mortality should always be less than 40% in control mice. Thus, at an electroshock stimulus of 60 Hz, 50 mA and 0.2 seconds duration, the order of convulsive components and the percentage of control animals displaying the behaviors should be as follows: tonic flexion (100%), tonic extension (100%) and clonus (100%) with less than 40% mortality.

For testing compounds, the abolition of the tonic extensor component was the endpoint. Animals were dosed orally (PO) with either vehicle or test drug and at a specified time were given a maximal electric shock through corneal electrodes blotted with saline (as described above). A minimum of 10 animals were used per group and the percentage of animals in the group without tonic hindlimb extension recorded. Determination of an ED₅₀ dose (that dose which inhibits 50% of the tonic extension seizures) was made. The anticonvulsant activity of compound of formula **I** wherein R₁ and R₂ are hydrogen, R₃, R₄, R₅, and R₆ are methyl and X is carbon gave an ED₅₀ of 16 mg/kg in mice at 4 hours following oral dosing.

For treating epilepsy, a compound of formula **I** may be employed at a daily dosage in the range of about 10 to 2000 mg, usually in 1 to 4 daily divided doses, for an average adult human. A unit dose would contain about 5 to 500 mg of the active ingredient. This translates to a dose of about 0.1 to 30 mg/kg/day.

In general, compounds of formula **I** may be used in treating epilepsy in a manner similar to that used for phenytoin; e.g., orally administering a solid formulation twice/day. Medical aspects of the treatment of epilepsy are described in greater detail by L S. Goodman et al. in "The Pharmacological Basis of Therapeutics", 5th Ed. pages 201 to 226, Macmillan (1975).

The compounds of formula **I** preferably are administered in the form of a pharmaceutical composition. To prepare the pharmaceutical compositions of this invention, one or more sulfamate compounds of formula **I** are intimately admixed with a pharmaceutical carrier according to conventional pharmaceutical compounding techniques, which carrier may take a wide variety of forms depending on the form of preparation desired for administration, e.g., oral, by suppository, or parenteral. In preparing the compositions in oral dosage form, any of the usual pharmaceutical media may be employed. Thus, for liquid oral preparations, such as, for example, suspensions, elixirs and solutions, suitable carriers and additives include water, glycols, oils, alcohols, flavoring agents, preservatives, coloring agents and the like; for solid oral preparations such as, for example, powders, capsules and tablets, suitable carriers and additives include starches, sugars, diluents, granulating agents, lubricants, binders, disintegrating agents and the like. Because of their ease in administration, tablets and capsules represent the most advantageous oral dosage unit form, in which case solid pharmaceutical carriers are obviously employed. If desired, tablets may be sugar coated or enteric coated by standard techniques. Suppositories may be prepared, in which case cocoa butter could be used as the carrier. For parenterals, the carrier will usually comprise sterile water, though other ingredients, for purposes such as aiding solubility or for preservation, may be included. Injectable suspensions may also be prepared, in which case appropriate liquid carriers, suspending agents, and the like may be employed.

It is especially advantageous to formulate the aforementioned pharmaceutical compositions in unit dosage form for ease of administration and uniformity of dosage. The term "unit dosage form' as used in the specification and claims herein refers to physically discrete units suitable as unit dosages, each unit containing a predetermined quantity of active ingredient calculated to produce the desired therapeutic effect in association with the required pharmaceutical carrier.

The pharmaceutical compositions herein will contain, per unit dosage, e.g.. tablet, capsule, powder, injection, teaspoonful, suppository and the like. The compositions will be administrated in amounts as previously described herein with regard to the active ingredient and to the condition being treated. The dosages, however, may be varied depending upon the requirement of the patient, the severity of the condition being treated, and the compound being employed. Determination of optimum dosages for a particular situation is within the skill of the art.
In the following Example and throughout the specification the following terms and abbreviations are used: g (grams); mL (milliliters); min (minutes); h (hours); mol (moles); mmol (millimoles); M (molar); v/v (volume to volume): TLC (thin layer chromatography); HPLC (high pressure liquid chromato - graphy); C, H, N, etc. (the chemical symbols for the elements); Anal. Calcd. (analysis calculated): [α]_{D}²⁵ (specific rotation measured at 25 °C with 589 nanometer light); c (concentration in grams per 100 mL); ¹H NMR (proton nuclear magnetic resonance spectrum); NMR abbreviations: s = singlet, d = doublet, t =triplet, m = multiplet, br = broadened, dd = doublet of doublets; Cl-MS (chemical ionization mass spectrum); mp (melting point). All melting points are corrected.

### Example 1 : (1R,2R,3S,4S)-[1,2:3,4-Di-O-(1-methylethylidene)cyclohexan -1.2.3.4-tetraol-4-yl]methyl sulfamate. [VIII (R₁=R₂, = H:R₃R₄,R₅,R₆=Me)].

(1R,2R,3S)2,3-O-(1-Methylethylidene)-5-methoxycarbonyl-4-cyclohexen-1,2,3-triol (1.56 g, 6.8 mmol; prepared according to the method of Shing, T.K and Tang, Y. *Tetrahedron* **1990**, *46*, 6575-6584), pyridine (2.16 g, 27 mmol) and a catalytic amount of 4-dimethylaminopyridine were dissolved in methylene chloride (35 mL) and at 23°C phenyl chlorothionoformate (1.77 g, 10.2 mmol) was added slowly under argon. After 2 h, the reaction was poured into saturated ammonium chloride (200 mL) and diluted with methylene chloride. The organic layer was separated, washed with brine, dried (MgSO₄), and evaporated in vacuo to a yellow oil, which was purified by preparative HPLC (ethyl acetate/hexane, 1:8) to afford a white solid. Recrystallization from methylene chloride/2-propanol gave analytically pure white solid thionocarbonate **III** (R₅= R₆ = Me): mp 123-125 °C; CI-MS (CH₄) MH⁺ = 365; ¹H NMR δ1.42 (s. CH₃),1.45 (s, CH₃), 2.77 (m, 1 H, H₆ₐ), 3.00 (dd, J = 16.5, 5.5 Hz, H₆ₑ), 3.80 (s, OCH₃), 4.67 (br d, J = 3.4 Hz, H₃), 4.85 (br m, 1 H, H₂), 5.60 (ddd, J =10.1, 5.5, 2.3 Hz, H₁), 6.81 (m, 1.0 H, H₄), 7.13 (dd, 2H. J = 7.4,1.2 Hz, ortho arom.), 7.31 (dd, 1 H, J = 7.3, 7.4 Hz, para arom.). 7.43 (dd, 2 H, J = 7.4, 8.0 Hz, meta arom.); [α]_{D}²⁰ -6.07 (c = 0.692, CH₃OH). Anal. Calcd for C₁₈H₂₀O₆S: C, 59.33; H, 5.53. Found: C, 59.22; H, 5.48.

Thionocarbonate **III** (R₅ = R₆ = Me; 1.53 g, 4.2 mmol), tributyltin hydride (1.83 g, 6.3 mmol), and tert-butyl peroxide (123 mg, 0.84 mmol) were combined in toluene (80 mL) and heated at reflux for 1.5 h. Upon cooling, the reaction was evaporated in vacuo to a clear oil, which was dissolved in diethyl ether (100 mL). The ether solution was washed once with 1 M NaOH (100 mL), water (50 mL), brine (50 mL), dried (MgSO₄), and evaporated in vacuo to give crude product. This was purified by preparative HPLC (ethyl acetate/hexane, 1:8) to afford ester **IV** (R₅ = R₆ = Me) as a clear oil: CI-MS (CH₄) MH⁺= 213; ¹H NMR δ 1.37 (s, CH₃), 1.39 (s, CH₃), 1.80 (m, 1H, H₆ₐ), 2.05 (m, 1H, H₅ₐ), 2.25-2.45 (m, 2H. H₅ₑ/H₆ₑ), 3.76 (s. OCH₃), 4.35 (ddd, J = 3.0, 5.4, 5.4 Hz, H₁), 4.59 (m, H₂), 6.78 (br s, 0.95 H, H₃).

The ester **IV** (R₅ = R₆ = Me; 0.66 g, 3.1 mmol) was combined with trimethylamine oxide dihydrate (0.49 g, 4.40 mmol), pyridine (1.50 g, 18.8 mmol), and water (0.30 g, 16.8 mmol) in tert-butanol (30 mL) and osmium tetroxide (30 mg, 0.12 mmol) was then added at 23°C. The reaction was heated at reflux for 2 h, cooled, and diluted with 20% NaHSO₃ (15 mL), and stirred for 30 min at 23°C. The solution was evaporated in vacuo to a residue, which was partitioned between water and ethyl acetate. The organic extract was washed with brine, dried (MgSO₄), and evaporated in vacuo to give a light yellow oil, diol V (R₅ = R₆ = Me): CI-MS (CH₄) MH⁺ = 247; ¹H NMR δ 1.39 (s, CH₃), 1.55 (s, CH₃), 1.65 (m, 1H, H₆ₐ), 2.0-2.2 (m, 3H, H₆ₑ/H₅ₐ/H₅ₑ), 2.28 (d, J = 7.2 Hz, OH), 3.28 (s, OH), 3.88 (dd, J = 7.3, 7.5 Hz, H₃), 3.98 (dd, J = 5.1, 7.7 Hz, H₂), 4.45 (m, 1H. H₁).

2-Methoxypropene (0.26 g, 3.6 mmol) was added to the diol **V** (R₅ = R₆ = Me; 0.44 g, 1.8 mmol) in methylene chloride (10 mL) under argon, followed by a catalytic amount of camphorsulfonic acid. After 1.5 h, another equivalent of 2-methoxypropene (0.13 g. 1.8 mmol) was added and stirring was continued for 1 h. Saturated NaHCO₃ (5 mL) was added and the organic phase was separated, washed with brine, dried (MgSO₄), and evaporated in vacuo to give crude bis-acetonide **VI** (R_{**3**}**,** R₄, R₅, R₆ = Me), as an oil: CI-MS (CH₄) MH⁺ = 287; ¹H NMR δ1.34 (s, CH₃), 1.35 (s, CH₃), 1.47 (s, CH₃), 1.48 (s, CH₃), 1.60-1.90 (m, 3H, H₅ₑ/H₆ₑ/H₆ₐ), 2.00 (ddd, J = 5.3, 15.0, 15.0 Hz, H₅ₐ), 3.80 (s, OCH₃), 4.43 (m, H₁), 4.50 (dd, J = 2.2, 7.6 Hz, H₂), 4.71 (d, J = 2.2 Hz, H₃).

This bis-acetonide **VI** (R₃, R₄, R₅, R₆ = Me; 390 mg, 1.36 mmol) in tetrahydrofuran (7.0 mL) was cooled to -20°C (ice/methanol bath) and diisobutylaluminum hydride (DIBAL-H, 2.75 mL, 1 M in THF) was added over 15 min and the reaction was allowed to come to 23°C. After 30 min, the reaction was cooled again to -10°C and another portion of DIBAL-H (2.75 mL) was added. When the reaction was complete (TLC), saturated ammonium chloride (10 mL) was added to the ice-cooled reaction and it was stirred for 10 min. The mixture was evaporated in vacuo and the residue was extracted three times with chloroform (50 mL). The organic phase was washed with brine, dried (MgSO₄) and evaporated in vacuo to a clear oil. This was purified by preparative TLC (ethyl acetate/hexane, 1:2) to afford the alcohol **VII** (R₃, R₄, R₅, R₆ = Me), as a clear viscous oil: CI-MS (CH₄) MH⁺ = 259; ¹H NMR δ 1.34 (s, CH₃), 1.40 (s, CH₃), 1.46 (s, CH₃), 1.47 (s, CH₃), 1.60-1.95 (m, 4 aliphatics), 2.08 (dd, J = 5.7. 7.2 Hz, OH), 3.55 (m, 2H, CH₂O), 4.27 (d, J = 2.7 Hz, H₃), 4.44 (m, 1H, H₁), 4.54 (dd, J = 2.7, 7.5 Hz, H₂).

The alcohol **VII** (R₃, R₄, R₅, R₆ = Me; 0.24 g, 0.93 mmol) and triethylamine (0.20 g, 2 mmol) were combined in dimethytformamide (6 mL) and cooled to 0°C under argon. Sulfamoyl chloride (0.215 g, 1.86 mmol) was added and the reaction stirred for 2 h at 0°C, whereupon additional triethylamine (0.20 g, 2 mmol) and sulfamoyl chloride (0.215 g, 1.86 mmol) were added; stirring was continued for 1 h at 0°C. The reaction was partitioned between methylene chloride and dilute NaHCO₃ and the organic phase was separated and washed three times with water, once with brine, dried (MgSO₄), and evaporated in vacuo to give a viscous oil. The product was purified by preparative TLC (ethyl acetate/hexane, 1:2) to afford viscous oil sulfamate **VIII** (R₁ = R₂ = H; R₃, R₄**,** R₅, R₆ = Me): CI-MS (NH₃) MH⁺ = 338; ¹H NMR δ 1.34 (s, CH₃), 1.44 (s, CH₃), 1.45 (s, CH₃), 1.47 (s. CH₃), 1.60-1.90 (m, 4 aliphatics), 4.16 (d, J = 11.1 Hz, CHₐOSO₂), 4.21 (d**,** J = 2.6 Hz, H₃), 4.29 (d, J = 11.1 Hz. CH_{b}OSO₂). 4.44 (br d, J = 7.5 Hz, H₁), 4.53 (dd, J = 2.6, 7.5 Hz, H₂), 4.95 (br s, NH₂); [α]_{D}²⁵ +1.20 (c = 0.5. CH₃OH). Anal. Calod for C₁₃H₂₃NO₇S: C, 46.28; H, 6.87; N, 4.15. Found: C, 46.08; H, 6.89; N, 4.19.

## Claims

1. A compound represented by the formula I: 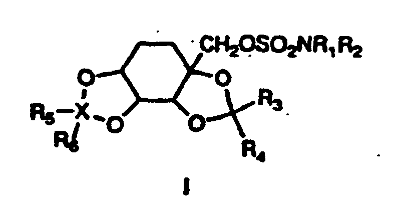
wherein:
X is carbon;
R₁ and R₂ are each hydrogen; and
R₃, R₄, R₅ and R₆ are each methyl; or
the pharmaceutically acceptable salt, hydrate, anomer, diastereomer, or enantiomer thereof.

2. The compound of claim 1, being
(1R,2S,3S,4S)-(1,2:3,4-di-O-methyethylidenecyclohexan-1,2,3,4-tetraol-4-yl)methyl sulfamate.

3. A pharmaceutical composition comprising the compound of claim 1, in combination with a pharmaceutically acceptable carrier, said compound being present in a therapeutically effective amount for treating convulsions.

4. A compound of claim 1 for use in a method for the treatment of convulsions.

5. A compound of claim 1 for use in the manufacture of a medicament for the treatment of convulsions.

## Revendications

1. Composé représenté par la formule I: 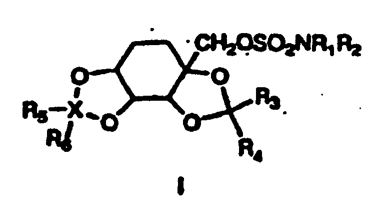
où:
X est carbone;
R₁ et R₂ sont chacun hydrogène; et
R₃, R₄, R₅ et R₆ sont chacun méthyle; ou
son sel, hydrate, anomère, diastéréomère, ou énantiomère pharmaceutiquement acceptable.

2. Composé de la revendication 1, qui est
(1R, 2R, 3S, 4S)-(1,2:3,4-di-O-méthyléthylidènecyclohexan-1,2,3,4-tétraol-4-yl)méthyl sulfamate.

3. Composition pharmaceutique comprenant le composé de la revendication 1, en combinaison avec un support pharmaceutiquement acceptable, ledit composé étant présent en une quantité thérapeutiquement efficace pour le traitement des convulsions.

4. Composé de la revendication 1 à utiliser dans une méthode pour le traitement des convulsions.

5. Composé de la revendication 1 à utiliser dans la fabrication d'un médicament pour le traitement des convulsions.

## Patentansprüche

1. Eine Verbindung dargestellt durch Formel I: 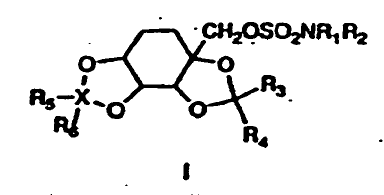
wobei:
X Kohlenstoff ist;
R₁ und R₂ sind jeweils Wasserstoff; und
R₃, R₄, R₅ und R₆ sind jeweils Methyl; oder
das pharmazeutisch akzeptable Salz, Hydrat, Anomer, Diastereomer oder Enantiomer davon.

2. Die Verbindung nach Anspruch 1, die
(1R,2R,3S,4S)-(1,2:3,4-di-O-Methylethylidencyclohexan-1,2,3,4-tetraol-4-yl)methylsulfamat ist.

3. Pharmazeutische Zusammensetzung, umfassend die Verbindung nach Anspruch 1 in Kombination mit einem pharmazeutisch akzeptablen Träger, wobei die Verbindung in einer therapeutisch effektiven Menge zur Behandlung von Krämpfen vorhanden ist.

4. Verbindung nach Anspruch 1 zur Verwendung in einem Verfahren zur Behandlung von Krämpfen.

5. Verbindung nach Anspruch 1 zur Verwendung bei der Herstellung eines Medikaments zur Behandlung von Krämpfen.
